# EUROPÄISCHE PATENTANMELDUNG

(11) **EP 1 842 569 A2**
(43) Veröffentlichungstag der Anmeldung: **10.10.2007**
(21) Anmeldenummer: 07113417.5
(22) Anmeldetag: 18.02.2002
(51) Int. Cl.: A61L 31/16

(54) **Implantate mit FK506**

(30) Priorität: 16.02.2001 DE 10107339; 05.06.2001 DE 10127011; 06.06.2001 DE 10127330
(62) Teilanmeldung aus: 02700248.4
(71) Anmelder: Abbott Laboratories Vascular Enterprises Limited, Terrace, Dublin 2 (IE); Astellas Pharma Inc., Chuo-ku, Tokyo (JP)
(72) Erfinder: Wnendt, Stephan, 52066, Aachen (DE); Von Oepen, Randolf, 72074, Tübingen (DE); Kuttler, Bernd, 72770, Reutlingen (DE); Lang, Gerhard, 72172, Sulz (DE)
(74) Vertreter: Peters, Hajo

(57) **Zusammenfassung**

Die Erfindung betrifft Implantate, insbesondere intrakavernöse oder intravaskuläre, vorzugsweise zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose, die in chemisch kovalent oder nicht-kovalent gebundener oder physikalisch fixierter Form FK506 enthalten, Verfahren zu deren Herstellung und deren Verwendung.

## Beschreibung

Die Erfindung betrifft Implantate, insbesondere intrakavernöse oder intravaskuläre, vorzugsweise zur Behandlung oder Prophylaxe von koronaren oder peripheren Gefäßverschlüssen oder Gefäßverengungen, insbesondere von Verengungen bzw. Stenosen oder Restenosen, vorzugsweise zur Prophylaxe von Restenose, die in chemisch kovalent oder nicht-kovalent gebundener oder physikalisch fixierter Form FK506 enthalten, Verfahren zu deren Herstellung und deren Verwendung.

Die Bildung arteriosklerotischer Lesionen in den arteriellen Blutgefäßen ist die zugrundeliegende Krankheit für eine große Bandbreite klinischer Symptome, die von Angina pectoris über Claudicatio intermittens zum Myocardinfarkt und dem ischämischen Schlaganfall reichen; alle basierend auf atheromer Bildung und/oder stenotischen Lesionen. Der Begriff stenotische Lesionen bezeichnet die lokale Reduktion des Gefäßlumens auf weniger als 60-70 % seines normalen Durchmessers, was wiederum zu einer deutlichen Reduktion der Versorgung des jeweiligen Gewebes mit Sauerstoff und Nährstoffen führt. Obgleich die Pharmakotherapie (Statine, ACE-Inhibitoren, gplla/IIIb Blocker und Plasminogen-Aktivatoren) insbesondere im Bereich cardiovaskulärer Krankheiten innerhalb der letzten Jahrzehnte gute therapeutische Erfolge gezeitigt hat, sind immer noch chirurgische Eingriffe (Bypass-Operationen etc.) bei vielen Patienten notwendig, die ein komplettes ischämisches Krankheitsbild entwickelt haben. Im übrigen sind diese Operationen relativ kompliziert und kostenintensiv und beinhalten das Risiko schwerer Komplikationen.

Um das Entstehen ischämischer Herzkrankheiten zu verhindern, sind minimal invasive chirurgische Vefahren entwickelt worden. Die Erfindung perkutaner transluminaler Herzkranz-Angioplastie (Percutaneous Transluminal Coronary Angioplasty/PTCA) in den späten 70er Jahren war ein großer Durchbruch in der Cardiologie. Bei der PTCA werden aufblasbare Ballons verwendet, die bis zur stenotischen Lesion in den Koronar-Arterien vorgeschoben werden. Diese Ballons werden dann an den jeweiligen Zielpositionen aufgeblasen und erreichen eine Dilatation der stenotischen Region. Ein ähnliches Vorgehen kann auch bei der Dilatation der carotischen oder peripheren Arterien angewandt werden.

Trotzdem wurde relativ bald festgestellt, daß sich bei einem relativ großen Anteil der PTCA-Patienten an den Stellen, die mit dem Ballonkatheter dilatiert worden waren, eine wiederauftretende Stenose entwickelte. Es wurde dabei entdeckt, daß diese sogenannte Restenose durch eine Neubildung der vaskulären Architekur der Gewebeschichten entsteht. Die Einführung röhrenförmiger vaskulärer Metallimplantate, sogenannter Stents, bei der transluminalen Behandlung der Stenose verbesserte die Situation. In klinischen Studien (Serruys et al., N. Engl. J. Med. 331 (1994) 489-495) wurde nachgewiesen, daß die Anwendung von Stents an den Ballon-dilatierten Stellen das Auftreten der Restenose von ca. 45 % auf ca. 30 % zu senken vermochte. Obgleich dies bereits als signifikante Verbesserung bei der Verhinderung residualer Restenose anzusehen ist, gibt es immer noch einen deutlichen Anreiz für therapeutische Verbesserungen.

Bei detaillierten Studien der Pathophysiologie der Restenose im Stent wurde entdeckt, daß diese sich von der PTCA-induzierten Restenose unterscheidet. Entzündungsreaktionen, Hyperproliferation und die Einwanderung glatter Muskelzellen (smooth muscle cells (SMCs)) sind wichtige Faktoren der Neointima-Bildung, die zur Restenose im Stent führen. Es wurde im Tier-Restenose-Modell und selbst im menschlichen Gewebe festgestellt, daß die Hyperproliferation der SMC's mit Infiltration der Gewebe um die Aussteifungen des Stents durch Makrophagen und T-Zellen (Grewe et al., J. Am. Coli. Cardiol. 35 (2000) 157-63) einhergeht. In Analogie zu anderen klinischen Indikationen, bei denen Entzündungsreaktionen und Hyperproliferation von Zellen eine Rolle spielen und die durch medikamentöse Behandlung kontrolliert werden können, wurde auch versucht, die Restenose durch Pharmakotherapie zu behandeln. Ausgewählte Wirkstoffe wurden entweder oral oder intravenös gegeben oder durch perforierte Katheter an den Wirkort gebracht. Unglücklicherweise vermochte bis dato keiner dieser Wirkstoffe die Restenose entscheidend zu reduzieren (Gruberg et al., Exp. Opin. Invest. Drugs 9 (2000) 2555-2578).

Die direkte Abgabe von pharmakologisch aktiven Wirkstoffen von mit Wirkstoff beschichteten Stents ist hier die Methode der Wahl. Tierversuche und erste Ergebnisse klinischer Versuche mit mit Wirkstoff beschichteten Stents erwecken den Anschein, daß eine verzögerte Freisetzung immunosuppressiver oder antiproliferativer Wirkstoffe das Risiko einer Restenose senken kann. Paclitaxel, ein cytostatischer Wirkstoff und Rapamycin, ein immunosuppressiver und cytostatischer Wirkstoff wurden in Tierversuchen getestet. Beide Verbindungen inhibieren die Neointima Bildung (Herdeg et al., Semin Intervent Cardiol 3 (1998) 197-199; Hunter et al., Adv. Drug. Delivery Rev. 26 (1997) 199-207; Burke et al., J Cardiovasc Pharmacol., 33 (1999) 829-835; Gallo et al., Circulation 99 (1999) 2164-2170). Trotzdem wurde nach 6 Monaten Implantation beschichteter Stents in Schweinen mit Paclitaxel eine Aufhebung des Effekts beobachtet (Heldman, International Local Drug Delivery Meeting and Cardiovascular Course on Radiation, Geneva, Jan 25-27, 2001). Rapamycin zeigte eine gute Effektivität mit einer kompletten Aufhebung der Restenose in ersten klinischen Anwendungen (Sousa et al., Circulation 103 (2001) 192-195). Andererseits scheint dies Hand in Hand mit einer verlangsamten Heilung der durch Ballon-Angioplastie und Stent-Platzierung verletzten Gefäßwand einher zu gehen.

Allgemein gesprochen ist ein Gleichgewicht zwischen Heilung der arteriellen Gefäßwand nach Angioplastie und Stent-Platzierung auf der einen Seite und der Eindämmung der Neointima-Bildung von großer Wichtigkeit. Um dieses Gleichgewicht zu erreichen, sollten Wirkstoffe verwendet werden, die selektiv mit spezifischen Mechanismen, die zur Neointima-Bildung führen, interferieren.

Daher war es Aufgabe der Erfindung Implantate mit günstigen Eigenschaften zur Behandlung und Prophylaxe von Restenose zur Verfügung zu stellen.

Gegenstand der Erfindung ist daher ein Implantat enthaltend in chemisch kovalent oder nicht-kovalent gebundener oder physikalisch fixierter Form FK506 sowie gegebenenfalls mindestens einen weiteren Wirkstoff.

Dabei gilt für jeden im Rahmen dieser Erfindung genannten Wirkstoff, inklusive des Wirkstoffes FK506, daß der Begriff Wirkstoff auch direkte Derivate des Wirkstoffes sowie den Wirkstoff auch in allen Arten von Salzen, Enantiomeren, Razematen, Basen oder freien Säuren des Wirkstoffes sowie Mischungen daraus beschreibt.

Bevorzugt ist es, wenn das Implantat ein intrakavernöses, vorzugsweise ein intravaskuläres Implantat ist.

Dabei versteht man unter intrakavernös innerhalb eines Hohlraums, insbesondere innerhalb eines Hohlorgans bzw. von Hohlorganen wie Blutgefäßen, Speiseröhren, Harnleiter, Gallengängen etc.

Unter intravaskulär ist insbesondere der Einsatz in einem Blutgefäß zu verstehen.

Bevorzugt ist es auch, wenn das Implantat zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose geeignet ist.

Besonders bevorzugt ist daher ein intrakavernöses, bevorzugt intravaskuläres Implantat zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose, enthaltend in chemisch kovalent oder nicht-kovalent gebundener oder physikalisch fixierter Form FK506 sowie gegebenenfalls mindestens einen weiteren Wirkstoff.

Das makrolide Antibiotikum FK506 (Tacrolimus, [3S-[3R*[E(1S*, 3S*, 4S*)], 4S*, 5R*,-8S*, 9E, 12R*, 14R*, 15S*, 16R*, 18S*, 19S*, 26aR*]]-5,6,8,11,12, 13, 14, 15, 16, 17, 18, 19, 24, 25, 25, 26, 26a-Hexadecahydro-5,19-dihydroxy-3-[2-(4-hydroxy-3-methoxycyclohexyl)-1-methylenthenyl]-14,16-dimethoxy-4,10,12,18-tetramethyl-8-(2-propenyl)-15,19-epoxy-3H-pyrido[2,1-c][1,4]oxaazacyclotricosine-1,7,-20,21(4H,23H)-tetrone;Merck index no. 9000.) ist ein für die Transplantationsmedizin entwickelter Wirkstoff. FK506 inhibiert die Abgabe von lnterleukin-2 (IL-2) und lnterferan-γ (IFN-γ) aus T-Zellen und blockt dabei die Abstoßung des Implantats (Grafts) (Wiederrecht et al., Ann. NY Acad Sci. 696 (1993) 9-19). FK506 wurde auch in SMC-Kulturen in Hinblick auf eine Inhibition der Proliferation der glatten Muskelzellen (Mohacsi et al., J. Heart Lung Transplant. 16 (1997) 484-492; Marx et al., Circulation Res., 76 (1995) 412-417) und ihrer Migration (Poon et al., J. Clin. Invest. 98 (1996) 2777-2283) untersucht. Generell wurde FK506 von verschiedenen Forschern wegen seiner geringen Potenz als ungeeignet für die Prävention der Restenose eingeschätzt (Mohacsi et al, (1997); Poon et al. (1996); Marx et al., 1995; Dell, Curr Med Chem 5 (1998) 179-94). Mohacsi et al. fanden eine halbmaximale Inhibition der SMC-Proliferation zwischen 100 nM and 1 µM, während Marx et al, bei Konzentrationen bis zu 123 nM gar keinen Effekt beobachteten. Im Gegensatz dazu ist Rapamycin bei der Inhibierung der Proliferation der SMC-Kulturen im nanomolaren Konzentrationsbereich aktiv.

Ausgehend von diesem Stand der Technik schien der Einsatz ausgerechnet von FK506 zur Hemmung der Restenose überhaupt nicht Erfolg versprechend (Mohacsi et al. (1997); Poon et al. (1996)). Überraschenderweise hatte es sich aber entgegen der Meinung der Fachwelt gezeigt, daß insbesondere der Einsatz von FK506 als Teil eines Stents aber auch anderer Implantate sehr effektiv in der Behandlung und Prophylaxe von Restenose ist. Gerade die lokale Applikation von FK506 ist günstig zur Verhinderung der Restenose und die Wirkung ist sehr ausbalanciert, denn sie erlaubt auch eine gute Re-Endothelialisierung der verletzten Gefäßwand.

Ohne daß dies von vorn herein anzunehmen war, könnte sich dies möglicherweise mit der immunomoduiatorischen Aktivität von FK506 erklären lassen, die sich in einer halbmaximalen Inhibition der IL-2-Freisetzung bei Konzentrationen um 0,1 nM (Kino et al., J. Antibiot. 40 (1987) 1256-1265) und einem inhibitorischen Effekt auf die SMC-Proliferation bei Konzentrationen um 300-500 nM zeigt. Entsprechend günstig ist daher der Einsatz von FK506.

Dabei versteht man unter Stenose den Verschluß oder die Verengung eines Gefäßes und unter Restenose das Wiederauftreten einer Stenose.

Weiter versteht man hier unter "enthaltend" unter anderem auch eine beispielsweise nicht kovalent gebundene Beschichtung.

Weiter bezieht sich hier "peripher" insbesondere auf Gefäße bzw. andere Hohlorgane außerhalb des Herzens und der Herzkranzgefäße.

Unter "chemisch nichtkovalent" gebunden sind insbesondere Bindungen durch Wechselwirkungen wie Wasserstoffbrücken, hydrophobe Wechselwirkungen, Van der Waals-Kräfte etc. zu verstehen.

Unter physikalisch fixiert ist beispielsweise das Einschließen z.B. durch eine Membran in einem Loch zu verstehen oder sterisches Entrapment durch Wahl der Öffnungsgrößen etc..

Unter Implantat ist jede Form eines künstlichen Objektes, das (auch nur zeitlich begrenzt) eingebracht wird, zu verstehen. Insbesondere handelt es sich dabei um intrakavernöse, beispielsweise intravaskuläre Implantate. Beispiele sind Stents, Grafts, Stent Grafts, Graft-Verbinder, Führungsdrähte, Katheterpumpen oder Katheter.

Unter Stent versteht man im Sinne dieser Erfindung ein längliches, im Inneren hohles Implantat mit mindestens zwei Öffnungen und meist kreisförmigem oder eliptischem aber auch beliebigem anderen Querschnitt (meist aus Metall aber gegebenenfalls auch aus Kunststoffmaterialien bzw. Polymeren) von bevorzugt durchbrochener, gitterförmiger Struktur, das in Gefäßen, insbesondere Blutgefäßen implantiert wird, um diese offen bzw. funktionsfähig zu halten.

Unter Graft versteht man im Sinne dieser Erfindung ein längliches, im Inneren hohles Implantat mit mindestens zwei Öffnungen und meist kreisförmigem oder eliptischem aber auch beliebigem anderen Querschnitt und mit mindestens einer homogenen oder gegebenenfalls aus verschiedenen Strängen gewebten, geschlossenen und für korpuskuläre Blutbestandteile und/oder für Wasser undurchlässigen Polymer-Oberfläche, das im allgemeinen als Gefäß-Prothese dient und meist bei beschädigten Gefäßen oder anstatt Gefäßen eingesetzt wird.

Unter Stent Graft versteht man im Sinne dieser Erfindung die Verbindung zwischen Stent und Graft. Entsprechend ist ein Stent Graft im Grunde eine mit einem Stent verstärkte Gefäß-Prothese (Graft s.o.), wobei die Polymerschicht homogen oder gegebenenfalls aus verschiedenen Strängen gewebt, geschlossen und für korpuskuläre Blutbestandteile und/oder für Wasser undurchlässig ist. Im engeren Sinne ist dies ein Stent, der auf mindestens 20% der Oberfläche des Implantats eine durchbrochene (gitterförmige), bevorzugt metallene Außenschicht und mindestens eine innerhalb oder außerhalb dieser Außenschicht liegende homogene oder gegebenenfalls aus verschiedenen Strängen gewebte, geschlossene und für korpuskuläre Blutbestandteile und/oder für Wasser undurchlässige Polymerschicht sowie gegebenenfalls (bei außenliegender durchbrochener Schicht) eine weitere innerhalb der Polymerschicht liegende, durchbrochene (gitterförmige) bevorzugt metallene Innenschicht oder eine außen und außerhalb der durchbrochenen Schicht liegende homogene oder gegebenenfalls aus verschiedenen Strängen gewebte, geschlossene und für korpuskuläre Blutbestandteile und/oder für Wasser undurchlässige Polymerschicht aufweist.

Unter Graftverbinder versteht man im Sinne dieser Erfindung ein Implantat, daß mindestens zwei Hohlorgane, Gefäße oder Grafts verbindet, aus den für Grafts oder Stent Grafts definierten Materialien besteht und/oder den für diese definierten Aufbau hat und entsprechend mindestens zwei, vorzugsweise drei oder vier Öffnungen aufweist, insbesondere eine asymetrische "T"-Form zeigt.

Unter Katheter versteht man im Sinne dieser Erfindung ein röhrenförmiges Instrument zur Einführung in Hohlorgane. Im engeren, bevorzugten Sinne sind es Führungs-, Angiographie- oder Ballonkatheter.

Unter Katheterpumpe versteht man im Sinne dieser Erfindung einen an seiner Spitze mit einem Propeller versehenen Katheter, der das Pumpen des Herzmuskels unterstützen kann.

Es ist eine weitere bevorzugte Ausführungsform des erfindungsgemäßen Implantats, wenn das Implantat wenigstens eine aus einem Metall oder einer Metalllegierung bestehende, homogene oder aus verschiedenen Strängen gebildete, geschlossene oder durchbrochene Schicht oder Oberfläche aufweist.

Unter Metall bzw. Metall-Legierung im Sinne dieser Erfindung sind insbesondere Stahl bzw. Stahl-Legierungen aber auch Nickel bzw. Nickel-Legierungen zu verstehen, wobei der Begriff Metall bereits von vorneherein auch Metall-Legierungen mitumfaßt.

Unter durchbrochen sind insbesondere gitterförmige, gewebte oder geflochtene Strukturen zu verstehen.

Es ist eine weitere bevorzugte Ausführungsform des erfindungsgemäßen Implantats, wenn das Implantat wenigstens eine aus einem Polymer bestehende, homogene oder aus verschiedenen Strängen gebildete, geschlossene oder durchbrochene Schicht oder Oberfläche aufweist.

Bei einer bevorzugten Ausführungsform weist das Implantat wenigstens eine Polymerschicht auf, die ganz oder teilweise eine aus einem Metall oder einer Metalllegierung bestehende, homogene oder aus verschiedenen Strängen gebildete, geschlossene oder durchbrochene Schicht oder Oberfläche, vorzugsweise eine aus einem Metall oder einer Metalllegierung bestehende, gegebenenfalls gitterförmige Struktur, bedeckt.

Bei einer besonders bevorzugten Ausführungsform weist das Implantat wenigstens eine aus einem Metall oder einer Metalllegierung bestehende, homogene oder aus verschiedenen Strängen gebildete, geschlossene oder durchbrochene Schicht oder Oberfläche und wenigstens eine aus einem Polymer bestehende, homogene oder aus verschiedenen Strängen gebildete, geschlossene oder durchbrochene Schicht oder Oberfläche auf.

Dabei ist es besonders bevorzugt, wenn bei diesem Implantat die aus einem Metall oder einer Metalllegierung bestehende Schicht oder Oberfläche eine aus einem Metall oder einer Metalllegierung bestehende, gegebenenfalls gitterförmige Struktur ist und/oder die aus einem Polymer bestehende Schicht oder Oberfläche homogen geschlossen oder gewebt ist und/oder wasser- und/oder korpuskelundurchlässig ist und/oder die Abfolge der Schichten und Oberflächen von außen nach innen Metall-Polymer, Polymer-Metall, Metall-Polymer-Metall oder Polymer-Metall-Polymer ist und/oder entweder die aus einem Polymer bestehende Schicht oder Oberfläche nicht chemisch (kovalent oder nichtkovalent) mit der aus einem Metall oder einer Metalllegierung bestehenden Schicht oder Oberfläche verbunden ist oder die aus einem Polymer bestehende Schicht oder Oberfläche mittels eines Klebstoffes mit der aus einem Metall oder einer Metalllegierung bestehenden Schicht oder Oberfläche verbunden ist.

Es ist weiter bevorzugt, wenn das im Rahmen der Implantate verwendete Polymer ausgewählt ist aus Dacron; Polytetrafluorethylen (PTFE/Teflon), ausdehnbar oder nicht ausdehnbar; oder Polyurethan; vorzugsweise aus Polytetrafluorethylen (PTFE), ausdehnbar oder nicht ausdehnbar; oder Polyurethan; insbesondere aus PTFE.

Es ist auch eine bevorzugte Ausführungsform der Erfindung, wenn das Implantat ein Stent, ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft ist.

Es ist besonders bevorzugt, wenn das erfindungsgemäße Implantat mit FK506 beschichtet ist.

Eine lokale Anwendung von FK506 wird durch direkte Abgabe von der wirkstoffbeladenen Oberfläche eines koronaren oder peripheren Stents erreicht. Eine wirkstoffbeladene Oberfläche eines Stents kann durch Verwendung verschiedener technologischer Ansätze erreicht werden. Jeder dieser Ansätze kann so durchgeführt werden, daß der Wirkstoff von der Oberfläche entweder in einem kurzen (Stunden) oder einem ausgedehnten Zeitraum (Tage) freigesetzt wird. Die Freisetzungskinetik kann dadurch angepaßt werden, daß spezifische Modifikationen an der Oberfläche vorgenommen werden, z.B. hydrophobe oder hydrophile Seitenketten eines polymeren Trägers oder eine keramische Oberfläche. Auch diese Oberflächen können ihrerseits wieder an der Oberfläche modifiziert werden, z.b. durch Si-Gruppen auf einer Aluminiumoxid-Schicht.

Die Freisetzungskinetik kann dadurch angepasst werden, dass spezifische Polymere, Blockpolymere, Polymerblends, Pfropfpolymere alleine oder im Schichtaufbau eingesetzt werden. Besonders geeignet zur Steuerung der Freisetzungskinetik ist die Verwendung von Nanokapseln und/oder Liposomen und den oben beschriebenen Polymerkombinationen. Unter Nanokapseln versteht man im allgemeinen das Umhüllen von mizellaren Systemen oder kolloidalen Feststoffen zu ultrafeinen Partikeln mit einem festen Überzug. Die umhüllten Teilchen, deren Größe im Nanometerbereich liegt, lösen sich kolloidal. Nanoverkapselte Wirkstoffe können so mit verlängerter Wirksamkeit eingesetzt werden. Liposomen werden im allgemeinen aus Phospholipiden durch Dispergieren in wäßrigen Medien gebildet und sind in diesem Zusammenhang interessant, da hydrophile Wirkstoffe in das wäßrige Innenvolumen wie auch in die wäßrigen Zwischenschichten und hydrophobe Wirkstoffe in die Lipidschichten eingebaut werden können. Verwendet man Nanokapseln und/oder Liposomen In unterschiedlicher Zusammensetzung so können diese mit verschiedenen Wirkstoffen beladen werden und so eine Wirkstoffkombination gezielt freigesetzt werden.

### Keramische Beschichtung

Eine Aluminiumoxid-Beschichtung (Patentanmeldungen DE 19855421, DE 19910188, WO 00/25841) mit poröser Oberfläche kann mit FK506 mit Mengen zwischen 10 µg und 10 mg entweder durch Eintauchen, Aufsprühen oder einer vergleichbaren Technik beladen werden. Die Dosis des Wirkstoffes hängt von der Art des Zielgefäßes und dem Zustand des Patienten ab und wird so gewählt, daß Proliferation, Migration und T-Zell-Antwort ausreichend inhibiert werden, ohne daß der Heilungsprozeß behindert wird. FK506 kann als wässrige oder organische Lösung, beispielsweise in DMSO, DMF und Ethanol verwendet werden. Nach dem Sprühen oder Eintauchen (gegebenfalls unter schwachem Vakuum) wird der behandelte Stent getrocknet und der Vorgang 2-10-mal wiederholt. Eine andere Möglichkeit in der Aufbringung besteht in der direkten Aufgabe der Wirkstofflösung auf die Stent-Stränge mit Hilfe einer Mikropipette oder eines Pipettierroboters. Nach dem letzten Trockenschritt kann der Stent eine Minute lang bei Raumtemperatur in Wasser oder isotonischer Saline gespült und anschließend wieder getrocknet werden. Der Wirkstoffgehalt kann nach Herauslösen des Wirkstoffs mit einem geeigneten Lösungsmittel durch Standardmethoden (HPLC, LC-MS) analysiert werden. Freisetzungskinetiken können unter Verwendung einer Standard-Freisetzungsmeßapparatur gemessen werden. Der keramische Ansatz kann mit einer polymeren (gegebenenfalls bioabbaubaren) Beschichtung kombiniert werden.

Im Übrigen kann jede Art von Metalloxid-Beschichtungen analog eingesetzt werden, insbesondere zum Beispiel lridiumoxid wie im Patent US6,245,104 B1 beschrieben. Entsprechend ist jede Nennung von Aluminiumoxid im Folgenden so zu verstehen, daß darunter auch andere Metalloxide wie zum Beispiel Iridiumoxid zu verstehen sind.

### PTFE Membran: Stent Graft

Hier wird ein zu oben beschrieben vergleichbarer Ansatz verwendet. FK506 wird in den Vertiefungen der porösen PTFE-Membran eingelagert.

### Allgemeine polymerische Beschichtung

Verschiedene Polymere sind für eine Wirkstoffbeladung geeignet: Methacrylate-Polymere, Polyurethan-Beschichtungen, PTFE-Beschichtungen, Hydrogel-Beschichtungen, Der Wirkstoff kann entweder auf die End-Oberfläche aufgetragen werden (s.o.) oder wird direkt der Polymerisationslösung beigegeben. In den übrigen Details entspricht dieser technische Ansatz den oben bereits beschriebenen.

Verschiedene Polymere sowie Kombinationen dieser in Form von Polymerblends, Systeme mit Schichtaufbau, Blockcopolymer, Pfropfpolymere können verwendet werden. Geeignete Polymere sind: Acrylate- und Methacrylate, Silikone wie z.B. Polydimethylsiloxan, Polymethylenmalonester, Polyether, Polyester, bioresorbierbare Polymere, Polymere aus vinylischen Monomeren wie z.B. Polyvinylpyrrolidon und Vinylether, Poly-cis-1,4-butadien, Poly-cis-1,4-isopren, Poly-trans-1,4-isopren sowie vulkanisierte Produkte, Polyurethane, Polyharnstoffe, Polyamide, Polyimide, Polysulfone sowie Biopolymere wie z.B. Cellulose und ihre Derivate und Proteine und Fibrinkleber. Besonders interessante Eigenschaften zeigen Hydrogele welche wegen ihrer hohen Wasseraufnahme als äußerste Schicht (top coat) sehr gute Hämokompatibilität aufweisen. Hier können Hydrolgele wie z.B Polyacrylamid, Polyacrylsäure, Polymere mit Sauerstoff als Heteroatom in der Hauptkette wie z.B. Polyethylenoxid, Polypropylenoxid, Polytetrahydrofuran eingesetzt werden. Der Wirkstoff kann entweder auf die End-Oberfläche aufgetragen werden oder eingebettet in Nanokapseln und/oder Liposomen appliziert werden. Der Wirkstoff kann aber auch direkt in der Polymerisationslösung oder in der Polymerlösung enthalten sein. Bei einigen Polymer/Wirkstoffsystemen kann der Wirkstoff durch Quellen immobilisiert werden.

### Mechanischer Ansatz

Der mechanische Ansatz beruht auf Vertiefungen, die in die Stent-Streben mittels eines Lasers eingebracht werden. Diese Vertiefungen können dann mit FK506 gefüllt werden. Der mechanische (Vertiefungen)-Ansatz kann mit einer polymeren, gegebenenfalls bioabbaubaren Beschichtung kombiniert werden, die selbst wirkstoffbeladen ist. Nach einer anfänglichen Freisetzung aus der polymeren Beschichtung kann aus den wirkstoffgefüllten Vertiefungen langfristig Wirkstoff freigesetzt werden. In den übrigen Details entspricht dieser technische Ansatz den oben bereits beschriebenen.

Entsprechend ist es eine weitere bevorzugte Ausführungsform des erfindungsgemäßen Implantats, wenn das Implantat einen keramischen Überzug, insbesondere aus Aluminiumoxid, aufweist, an den FK506 gebunden ist.

Es ist eine weitere bevorzugte Ausführungsform des erfindungsgemäßen Implantats, wenn das Implantat einen polymeren Überzug, insbesondere aus Methacrylat-Polymeren, Polyurethan, PTFE, Hydrogel oder Hydrogel/Polyurethan-Blend, insbesondere PTFE aufweist, an den FK506 gebunden ist oder in dem FK506 vor Aufbringung des Überzugs gelöst wurde.

Es ist eine weitere bevorzugte Ausführungsform des erfindungsgemäßen Implantats, wenn das Metall des Implantats mittels Laser eingebrachte Vertiefungen aufweist, die mit FK506 gefüllt sind. Dabei ist es besonders günstig, wenn das mit FK506-gefüllten Vertiefungen versehene Metall oder mindestens die Vertiefungen mit einem biologisch abbaubaren Polymermaterial überzogen wird/werden, wobei gegebenenfalls FK506 an den polymeren Überzug gebunden wird oder FK506 vor der Polymerisation des Überzugs in dem Polymermaterial gelöst wurde.

Bei einer anderen sehr günstigen Ausführungsform des erfindungsgemäßen Implantats ist das Implantat herstellbar durch ein Verfahren, bei dem
a) ein wenigstens eine aus einem Metall oder einer Metall-Legierung bestehende, homogene oder aus verschiedenen Strängen gebildete, geschlossene oder durchbrochene Schicht oder Oberfläche aufweisendes Implantat gemäß einem der Ansprüche 4, 6 oder 7 bis 10, das mit einem keramischen Überzug, insbesondere aus Aluminiumoxid, überzogen wird, eingesetzt wird
   oder
b) ein wenigstens eine aus einem Polymer bestehende, homogene oder aus verschiedenen Strängen gebildete, geschlossene oder durchbrochene Schicht oder Oberfläche aufweisendes Implantat gemäß einem der Ansprüche 5 bis 10 eingesetzt wird,
   oder
c) ein Implantat gemäß einem der Ansprüche 1 bis 10, das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird, eingesetzt wird
   oder
d) ein Implantat gemäß einem der Ansprüche 4, 6 oder 7 bis 10 mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Implantat mit einem polymerisierten oder auf der Oberfläche polymerisierenden bioabbaubaren Überzug überzogen wird, eingesetzt wird,
e) dann das Implantat gemäß a), b), c) oder d) mit einer FK506-Lösung in wäßrigem oder organischem Lösungsmittel in Kontakt gebracht wird, beispielsweise durch Beträufeln, Besprühen oder Eintauchen gegebenenfalls unter Vakuum,
f) dann gegebenenfalls das Implantat getrocknet wird, vorzugsweise bis zur Entfernung des Lösungsmittels aus Schritt e),
g) dann gegebenenfalls Schritt e) gegebenenfalls gefolgt von Schritt f) wiederholt wird, vorzugsweise mehrfach, insbesondere 1 bis 5-fach, sowie
h) gegebenenfalls anschließend das Implantat ein- oder mehrfach mit Wasser oder isotoner Saline gespült wird und
i) gegebenenfalls anschließend getrocknet wird.

Dabei ist bevorzugt, wenn bei der Herstellung dieses so herstellbaren erfindungsgemäßen Implantats in Schritt e) FK506 in Alkohol gelöst wird, vorzugsweise in Ethanol, insbesondere in einer Konzentration von 0,5-5 g/l in FK506 in Ethanol und/oder in Schritt e) das Implantat unter Vakuum vorzugsweise über Nacht durch Eintauchen mit einer FK506-Lösung in wäßrigem oder organischem Lösungsmittel in Kontakt gebracht wird und/oder die Schritte f) und/oder g) nicht ausgeführt werden und/oder bei Schritt h) das Implantat mehrfach mit Saline gewaschen wird und/oder in Schritt i) das Implantat über Nacht getrocknet wird.

In einer alternativen besonders bevorzugten Ausführungsform der Erfindung ist bevorzugt, wenn bei der Herstellung dieses wie oben beschrieben herstellbaren erfindungsgemäßen Implantats in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird, ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die FK506-Lösung bewegt wird und abschließend, vorzugsweise nach Ablauf von ca. 12 h, das dünne, vorzugsweise steril, luftdurchlässige Belüftungsrohr entfernt wird und/oder in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im vorzugsweise sterilen verschlossenen Glasgefäß von Schritt e) verbleibt und/oder die Schritte f) bis i) unterbleiben.

Bei einer anderen sehr günstigen Ausführungsform des erfindungsgemäßen Implantats ist das Implantat herstellbar durch ein Verfahren, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

Es ist weiter besonders bevorzugt, wenn nach Implantierung des erfindungsgemäßen Implantats FK506 freigesetzt wird. Dabei ist besonders günstig, wenn die Freisetzung verzögert erfolgt. Dabei ist es eine besonders bevorzugte Ausführungsform der Erfindung, wenn FK506 vom lmplantat über einen Zeitraum von 24 h, vorzugsweise 48h, insbesondere mehr als 96h nach Implantierung freigesetzt wird. Insbesondere ist auch günstig, wenn das FK506 vom Implantat nach Implantation
a) innerhalb von < 48 h oder
b) über mindestens 48 h, vorzugsweise über mindestens 7 Tage, insbesondere über mindestens 2 bis zu 21 Tagen freigesetzt wird, oder daß
c) das Implantat beide Freisetzungsmuster a) und b) zeigt.

Gerade letztere Variante ist dann zu erreichen, wenn man zwei verschiedene Arten der Beschichtung, Bindung oder physikalischen Fixierung wählt. Ein Beispiel sind die mit FK506-beladenen bioabbaubaren Membranen versiegelten Laservertiefungen mit FK506. Nach rascher Freisetzung aus der Membran wird langfristig aus den Vertiefungen freigesetzt.

Es ist eine weitere bevorzugte Ausführungsform der Erfindung, wenn im Implantat mindestens ein weiterer Wirkstoff enthalten ist, vorzugsweise ein pharmazeutischer Wirkstoff, insbesondere einen weiteren Wirkstoff ausgewählt aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2212 (5-(Cyclopropyl-2-[1-(2-fluoro-benzyl)-1H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder anderen Corticosterioden; 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, lbuprofen, Naproxen oder anderen COX-9-lnhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK oder Interleukin-10;
(Gruppe 3:) Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol; Actinomycin D; Rheopro/Abciximab oder Probucol.

Dabei ist es besonders bevorzugt, daß wenn der weitere Wirkstoff ausgewählt ist aus Gruppe 1, dieser vom Implantat innerhalb der ersten 24 - 72 h nach Implantation freigesetzt wird und/oder, wenn der weitere Wirkstoff ausgewählt ist aus Gruppe 2, dieser vom Implantat innerhalb der ersten 48 h - 21 Tagen nach Implantation freigesetzt wird und/oder, wenn der weitere Wirkstoff ausgewählt ist aus Gruppe 3, dieser vom Implantat innerhalb von 14 Tagen bis 3 Monaten nach lmplantation freigesetzt wird.

Ein weiterer Gegenstand der Erfindung ist ein Verfahren zur Herstellung eines erfindungsgemäßen Implantats, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

Ein weiter Gegenstand der Erfindung ist ein Verfahren zur Herstellung eines erfindungsgemäßen Implantats mit folgenden Schritten:
a) ein wenigstens eine aus einem Metall oder einer Metall-Legierung bestehende, homogene oder aus verschiedenen Strängen gebildete, geschlossene oder durchbrochene Schicht oder Oberfläche aufweisendes Implantat gemäß einem der Ansprüche 4, 6 oder 7 bis 10, das mit einem keramischen Überzug, insbesondere aus Aluminiumoxid, überzogen wird,
   oder
b) ein wenigstens eine aus einem Polymer bestehende, homogene oder aus verschiedenen Strängen gebildete, geschlossene oder durchbrochene Schicht oder Oberfläche aufweisendes Implantat gemäß einem der Ansprüche 5 bis 10,
   oder
c) ein Implantat gemäß einem der Ansprüche 1 bis 10, das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird,
   oder
d) ein Implantat gemäß einem der Ansprüche 4, 6 oder 7 bis 10 mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Implantat mit einem polymerisierten oder auf der Oberfläche polymerisierenden vorzugsweise bioabbaubaren Überzug überzogen wird,
   wird eingesetzt,
e) dann wird das Implantat gemäß a), b), c) oder d) mit einer FK506-Lösung in wäßrigem oder organischem Lösungsmittel in Kontakt gebracht, beispielsweise durch Beträufeln, Sprühen oder Eintauchen, gegebenenfalls unter Vakuum,
f) dann wird gegebenenfalls das Implantat getrocknet, vorzugsweise bis das Lösungsmittel aus Schritt e) verdampft ist,
g) dann wird gegebenenfalls Schritt e) gegebenenfalls gefolgt von Schritt f), vorzugsweise mehrfach, insbesondere 1 bis 5-fach, wiederholt sowie
h) gegebenenfalls wird anschließend das Implantat ein- oder mehrfach mit Wasser oder isotoner Saline gespült und
i) gegebenenfalls wird anschließend getrocknet.

Besonders bevorzugt ist dieses Verfahren, wenn in Schritt e) FK506 in Alkohol gelöst wird, vorzugsweise in Ethanol, insbesondere in einer Konzentration von 0,5 - 5 g/l FK506 in Ethanol und/oder daß in Schritt e) das Implantat unter Vakuum vorzugsweise über Nacht durch Eintauchen mit einer FK506-Lösung in wäßrigem oder organischem Lösungsmittel in Kontakt gebracht wird und/oder daß die Schritte f) und/oder g) nicht ausgeführt werden und/oder daß bei Schritt h) das Implantat mehrfach mit Saline gewaschen wird und/oder daß in Schritt i) das Implantat über Nacht getrocknet wird.

Eine besonders bevorzugte Alternative des erfindungsgemäßen Verfahrens liegt vor, wenn in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird, ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die FK506-Lösung in Bewegung gehalten wird und abschließend vorzugsweise nach Ablauf von ca. 12h das dünne, vorzugsweise steril, luftdurchlässige Belüftungsrohr entfernt wird und/oder daß in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im sterilen verschlossenen Glasgefäß von Schritt e) verbleibt und/oder daß die Schritte f) bis i) unterbleiben.

Diese Verfahrensalternative ist besonders günstig, bisher völlig unbekannt und sowohl in den Kosten, als auch Herstellungszeit und -schritten äußerst vorteilhaft, zumal das Implantat sofort bereits steril verpackt entsteht. Es ist auch generell anwendbar und natürlich nicht auf FK506 beschränkt sondern funktioniert mit einer großen Vielzahl von Wirkstoffen. Gerade an solchen günstigen und einfachen Verfahren herrscht aber Mangel, so daß hier eine Aufgabe liegt.

Ein weiterer separater Gegenstand der Anmeldung ist daher ein im folgenden generelles genanntes Verfahren zur Herstellung mit Wirkstoffen beschichteter Implantate mit folgenden Schritten:
a) das Implantat wird, vorzugsweise steril, in ein vorzugsweise steriles Gefäß mit einem sich nach Ende einer Perforation verschließenden, perforierbarem Verschluß, beispielsweise in eine Injektions-Flasche eingebracht
b) vorzugsweise sterile Wirkstofflösung, vorzugsweise in einem organischen Lösungsmittel mit geringem Dampfdruck, insbesondere in Alkohol wie Ethanol oder Methanol, wird in das Gefäß eingebracht.
c) das Gefäß wird mit dem sich nach Ende einer Perforation verschließenden, perforierbarem Verschluß verschlossen,
d) ein dünnes, vorzugsweise steril, luftdurchlässiges Belüftungsrohr, beispielsweise eine Kanüle, wird perforierend durch den Verschluß gesteckt wird,
e) gegebenenfalls wird ein Vakuum angelegt, wobei vorzugsweise die Wirkstoff-Lösung bewegt wird,
f) abschließend, vorzugsweise nach Ablauf von ca. 12h, wird das dünne, vorzugsweise steril, luftdurchlässige Belüftungsrohr entfernt und
g) gegebenenfalls das Implantat bis zum Einsatz im vorzugsweise sterilen verschlossenen Glasgefäß von Schritt a) belassen.

Dabei ist für dieses generelle Verfahren günstig, wenn das Implantat von Schritt a) mindestens eine metallische durchbrochene oder geschlossene Oberfläche oder Schicht aufweist, eine keramische Beschichtung aufweist, eine polymere Beschichtung aufweist und/oder mindestens eine polymere, durchbrochene oder geschlossene Oberfläche oder Schicht aufweist.

Ebenso bevorzugt ist das generelle Verfahren für ein Implantat, das ein Stent, Stent Graft, Graft, Graft-Verbinder, Polymeroberflächen-Stent oder Katheter ist.

Bevorzugt ist das generelle Verfahren weiter, wenn der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors ;
(Gruppe 2:) Dexamethason, Betamethason, Prednison oder Corticosterioden; FK506 (Tacrolimus); 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol
insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); VEGF, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxal; Mitoxantron, Combretastatin A4; Flavopiridol.

Ein weiterer Gegenstand der Erfindung ist die Verwendung eines erfindungsgemäßen Implantats zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

Ein weiterer wichtiger Gegenstand der Anmeldung ist die im folgenden FK506-Verwendung genannte Verwendung von FK506 zur Beschichtung oder zur Herstellung eines Implantats zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

Bevorzugt ist es für die FK506-Verwendung, wenn das Implantat ein Stent, ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft bzw. ein Polymeroberflächen-Stent ist.

Weiter ist es für die FK506-Verwendungen günstig, wenn das FK506 am Implantat so gebunden oder angebracht ist, daß es nach Implantierung vom Implantat, vorzugsweise verzögert, freigesetzt wird.

Ein weiterer separater Gegenstand der Anmeldung ist die Verwendung von FK506 zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose. Wie oben ausgeführt hat hier FK506, wie im Rahmen dieser Anmeldung herausgefunden wurde, besonders günstige Eigenschaften.

Im Rahmen der Erfindung hat sich für FK506 besonders ein eine Polymerschicht aufweisender oder aus Polymeren bestehender Stent beziehungsweise ein Graft oder ein Stent Graft bewährt. Derartige Implantate, im Rahmen dieser Erfindung zusammenfassend als Polymeroberflächen-Stents bezeichnet, sind bisher noch nicht mit Wirkstoff-Beschichtung angewandt worden. Aber gerade dazu eignen sie sich ausweislich der im Rahmen dieser Anmeldung gemachten Untersuchungen hervorragend, da sie leicht mit Wirkstoff zu beladen sind und dann den Wirkstoff gleichmäßig und effektiv wieder abgeben. Diese Eigenschaft ist aber nicht auf FK506 beschränkt, so daß ein weiterer separater Gegenstand der Anmeldung ein Polymeroberflächen-Stent enthaltend in chemisch kovalent oder nicht-kovalent gebundener oder physikalisch fixierter Form mindestens einen physiologisch und/oder pharmazeutisch wirksamen Wirkstoff ist. Im Sinne dieser Erfindung versteht man unter Polymeroberflächen-Stent ein intravaskuläres Implantat im Sinne der Erfindung, das eine Polymeroberfläche aufweist. Im weiteren Sinne umfassen Polymeroberflächen-Stents damit Grafts sowie Stent Grafts, Graft-Verbinder, mit Polymeren beschichtete oder aus Polymeren bestehende Stents, im engeren Sinne mit Polymeren beschichtete oder aus Polymeren bestehende Stents, sowie Stent Grafts.

Für den Polymeroberflächen-Stent ist es bevorzugt, wenn der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine): Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol
insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1H-pyrazolo[3,4-n]pyridin-3-yl]-pyrimidin-4-ylamine); Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (TaGrolimus); VEGF, VEGF-Rezeptor Aktivatoren; Inhibitoren des Plasminogen Aktivators-1 (plasminogen activator inbibitors-1 oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Mitoxantron; Combretastatin A4; Flavopiridol;
und/oder daß der Polymeroberflächen-Stent mindestens zwei, vorzugsweise 2 oder 3 physiologisch und/oder pharmazeutisch wirksame Wirkstoffe ausgewählt aus einer der Gruppen 1 bis 3 enthält, vorzugsweise maximal einen Wirkstoff aus einer Gruppe.

Für eine weitere bevorzugte Ausführungsform dieses Polymeroberflächen-Stents gilt, daß, wenn der weitere Wirkstoff ausgewählt ist aus voranstehender Gruppe 1, dieser vom Implantat innerhalb der ersten 24 - 72 h nach Implantation freigesetzt wird und/oder, wenn der weitere Wirkstoff ausgewählt ist aus voranstehender Gruppe 2, dieser vom Implantat innerhalb der ersten 48 h - 21 Tagen nach Implantation freigesetzt wird und/oder, wenn der weitere Wirkstoff ausgewählt ist aus voranstehender Gruppe 3, dieser vom Implantat innerhalb von 14 Tagen bis 3 Monaten nach Implantation freigesetzt wird.

Generell gilt für erfindungsgemäße Polymeroberflächen-Stents, daß alle vorstehend beschriebenen Ausführungsformen und -arten, Herstellungsverfahren und Verwendungen des speziell FK506 enthaltenden Implantats auch für den erfindungsgemäßen Polymeroberflächen-Stent bevorzugt und damit Gegenstände dieser Erfindung sind, solange die Ausführungsformen noch Polymeroberflächen-Stents sind.

Ein weiterer Gegenstand der Erfindung ist auch die Behandlung eines Menschen oder Tieres, der oder das diese Behandlung benötigt, mit oder durch ein erfindungsgemäßes Implantat oder einen Polymeroberflächen-Stent.

Im folgenden Abschnitt wird die Erfindung weiter durch Beispiele erläutert, ohne sie darauf zu beschränken.

### Beispiele und Abbildungen:

### Abbildungen:

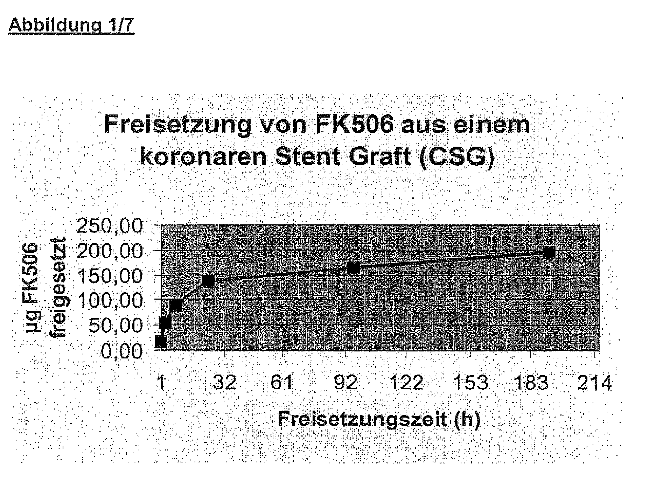

Abbildung 1 zeigt die Freisetzung von FK506 aus einem koronaren Stent Graft, bei dem die aus PTFE bestehende Oberfläche mit FK506 beladen wurde.

Abbildung 2 zeigt die Freisetzung von Candesartan und Quinapril aus einem Stent Graft, bei dem die aus PTFE bestehende Oberfläche mit Candesartan und Quinapril beschichtet wurde.

Abbildung 3 zeigt die Freisetzung von Candesartan und Quinapril aus einem mit Polyurethan beschichteten Stent, bei dem diese Beschichtung mit Candesartan und Quinapril angereichert wurde.

Abbildung 4 zeigt die Freisetzung von Candesartan und Quinapril aus einem mit Polyurethan/Hydrogel-Blend beschichteten Stent, bei dem diese Beschichtung mit Candesartan und Quinapril angereichert wurde.

Abbildung 5 zeigt die Freisetzung von FK506 im Blut nach Implantation entsprechend beschichteter Stents in Kaninchen.

Abbildung 6 zeigt die Fläche der Intima auf implantierten Stents mit und ohne entsprechender Beschichtung mit FK506.

Abbildung 7 zeigt die inflammatorische Reaktion auf Implantation von Stents mit und ohne entsprechender Beschichtung mit FK506.

### Beispiele:

### Beispiel 1:

### Beladung interessanter Verbindungen auf Stent Grafts

Alle Werte sind in µg angegeben.

**Tabelle 1**

| | | | | | |
|---|---|---|---|---|---|
| | | | | | |

| **Wirkstoff / Stent-Art** | **FK 506 (Tacrolimus)** | **Vinblastin** | **Paclitaxel (Taxol)** | **Cisplatin** | **Mitoxantron** |
|---|---|---|---|---|---|
| **A** | 1382 | 74 | 128 | 15* | 116 |
| | 1671 | 125 | 155 | 14* | 142 |
| | 1625 | 238 | 148 | 14* | 113 |
| **Durchschnitt** | **1559** | **146** | **144** | **14** | **124** |
| **B** | 18 | | | | |
| | 16 | | | | |
| | 21 | | | | |
| **Durchschnitt** | **18** | | | | |
| **C** | 194 | | | | |
| | 165 | | | | |
| **Durchschnitt** | **180** | | | | |

| | | | | | |
|---|---|---|---|---|---|
| * gemessen durch AAS | | | | | |

A: Versuche mit gelösten Feststoffen, bei denen Stent Grafts mit einer PTFE-Polymerschicht in die Lösung eingetaucht wurden.
B: Versuche mit i.v.-Lösungen, bei denen Stent Grafts mit einer PTFE-Polymerschicht in die Lösung eingetaucht wurden.
C: Versuche mit i.v.-Lösungen, bei denen mit Polyurethan beschichtete Stents in die Lösung eingetaucht wurden.

### Beispiel 2:

### Freisetzungsmuster verschieden hergestellter erfindungsgemäßer Stent Grafts wie auch allgemeiner Polymeroberflächen-Stents:

Alle Werte sind in µg angegeben.

Für die Analyse der Wirkstoff-Freisetzung wurde ein Stent bei 37°C in 10 ml PBS-Puffer (stabilisiert mit Na-Azid) at 37°C inkubiert. Nach Ablauf definierter Zeitabschnitte wurden 2 x 1 ml der Lösung entfernt und analysiert. Diese 2 ml wurden durch frischen PBS-Puffer (stabilisiert mit Na-Azid) ersetzt.

In den Tabellen ist der gesamte freigesetzte Gehalt an Wirkstoff in der Lösung angegeben. Das bedeutet, daß die Menge an Wirkstoff in dem für die Analyse entnommenen Puffervolumen zu der am nachfolgenden Zeitpunkt entnommenen Menge hinzuaddiert wurde.

**Tabelle 2:**

| **Vinblastin Wirkstoff/ Art** | **nach 1 h** | **nach 3 h** | **nach 8 h** | **nach 24 h** | **nach 96 h** **nach 72 h** |
|---|---|---|---|---|---|
| **A** | 73 und 75 Schnitt: **74** | 108 und 114 Schnitt: **109** | 121 und 126 Schnitt: **124** | 106 und 120 Schnitt: **113** | 132 und 140 (96h) Schnitt: **136** |
| | 37 und 41 Schnitt: **39** | 48 und 51 Schnitt: **50** | 47 und 58 Schnitt: **42** | 57 und 62 Schnitt: **60** | 56 und 57 (72h) Schnitt: **57** |
| | 80 und 86 Schnitt: **83** | 99 und 104 Schnitt: **102** | 108 und 117 Schnitt: **113** | 117 und 127 Schnitt: **122** | 113 und 121 (72h) Schnitt: **117** |

| | | | | | |
|---|---|---|---|---|---|
| A: Versuche mit gelösten Feststoffen, bei denen Stent Grafts mit einer PTFE-Polymerschicht in die Lösung eingetaucht wurden. | | | | | |

**Tabelle 3:**

| **FK 506 (Tacrolimus)** | **nach 1h** | **nach 3 h** | **nach 8 h** | **nach 24 h** | **nach 96 h** | **nach 192 h** |
|---|---|---|---|---|---|---|
| **Wirkstoff/ Art A 1. Stent** | 14 und 13 Schnitt: **14** | 62 und 62 Schnitt: **62** | 92 und 99 Schnitt: **95** | 148 und 145 Schnitt: **147** | 145 und 151 Schnitt: **148** | 195 und 198 Schnitt: **196** |
| | **nach 1 h** | **nach 3 h** | **nach 8 h** | **nach 24 h** | **nach 96 h** | |
| **A 2. Stent** | 34 und 26 Schnitt: **30** | 56 und 57 Schnitt: **57** | 82 und 78 Schnitt: **80** | 108 und 109 Schnitt: **109** | 159 und 164 Schnitt: **161** | |
| | | | | | | |
| | | | | | | |
| | **nach 1 h** | **nach 3 h** | **nach 8 h** | **nach 24 h** | **nach 96 h** | |
| **A 3. Stent** | 12 und 9 Schnitt: **11** | 47 und 43 Schnitt: **45** | 101 und 93 Schnitt: **95** | 154 und 155 Schnitt:**154** | 184 und 190 Schnitt:**187** | |

| | | | | | | |
|---|---|---|---|---|---|---|
| A: Versuche mit gelösten Feststoffen, bei denen Stent Grafts mit einer PTFE-Polymerschicht in die Lösung eingetaucht wurden. | | | | | | |

**Tabelle 4:**

| **FK 506 (Tacrolimus)** | **nach 1 h** | **nach 3 h** | **nach 8 h** | **nach 24 h** | **nach 96 h** | |
|---|---|---|---|---|---|---|
| **Wirkstoff/ Art** **C** **1. Stent** | 19 und 20 Schnitt: **20** | 25 und 26 Schnitt: **26** | 33 und 33 Schnitt: **33** | 41 und 39 Schnitt: **40** | 43 und 38 Schnitt: **41** | |
| | | | | | | |
| | | | | | | |
| **C** **2. Stent** | **nach 1 h** | **nach 3 h** | **nach 8 h** | **nach 24 h** | **nach 96 h** | **nach 264 h** |
| | 20 und 27 Schnitt: **24** | 21 und 24 Schnitt: **22** | 26 und 30 Schnitt: **28** | 34 und 31 Schnitt: **32** | 37 und 35 Schnitt : **36** | **85** |
| | **nach 367 h** | | | | | |
| | 88 und 94 Schnitt: **91** | | | | | |

| | | | | | | |
|---|---|---|---|---|---|---|
| C: Versuche mit i.v.-Lösungen, bei denen mit Polyurethan beschichtete Stents in die Lösung eingetaucht wurden. | | | | | | |

**Tabelle 5:**

| **Paclitaxel** | **nach 1 h** | **nach 3 h** | **nach 8 h** | **nach 24 h** | **nach 96 h** | **nach 192 h** |
|---|---|---|---|---|---|---|
| **Wirkstoff/Art** | | | | | | |
| **A** | 0.14 und 0.23 Schnitt: **0.19** | 0.46 und 0,53 Schnitt: **0.50** | 1.42 und 1.25 Schnitt: **1.34** | 1.65 und 1.42 Schnitt: **1.54** | 1.42 und 1.93 Schnitt: **1.68** | 2.22 und 2.24 Schnitt: **2.23** |
| | 0.42 und 0.52 Schnitt: **0.47** | 0.90 und 0.90 Schnitt: **0.90** | 1.16 und 1.21 Schnitt: **1.19** | 2.44 und 2.40 Schnitt: **2.42** | 2.79 und 2.18 Schnitt: **2.79** | |
| | 0.20 und 0.16 Schnitt: **0.18** | 0.51 und 0.95 Schnitt: **0.73** | 0.89 und 0.94 Schnitt: **0.92** | 2.26 und 2.27 Schnitt: 2**.27** | 2.82 und 2.82 Schnitt: **2.82** | |

| | | | | | | |
|---|---|---|---|---|---|---|
| A: Versuche mit gelösten Feststoffen, bei denen Stent Grafts mit einer PTFE-Polymerschicht in die Lösung eingetaucht wurden. | | | | | | |

**Tabelle 6:**

| **Cisplatin** | **nach 1 h** | **nach 3 h** | **nach 8 h** | **Nach 24 h** |
|---|---|---|---|---|
| **Wirkstoff/ Art** | | | | |
| **A** | 11.7 und 91.9 Schnitt: **11.8** | 15.4 und 15.4 Schnitt: **15.4** | 16.3 und 16.5 Schnitt: **16.4** | 16.1 und 15.9 Schnitt: **16.0** |
| | 5.7 und 5.4 Schnitt: 5.5 | 7.8 und 7.7 Scttnitt: 7.8 | 9.9 und 9.8 Schnitt: **9.8** | 10.9 und 11.1 Schnitt: 11.0 |
| | 10.0 und 10.0 Schnitt: **10.0** | 11.4 und 11.8 Schnitt: **11.6** | 12.2 und 12.3 Schnitt: **12.2** | 12.4 und 12.2 Schnitt: **12.3** |

| | | | | |
|---|---|---|---|---|
| A: Versuche mit gelösten Feststoffen, bei denen Stent Grafts mit einer PTFE-Polymerschicht in die Lösung eingetaucht wurden. | | | | |

**Tabelle 7:**

| **Mitoxantron** | **nach 1 h** | **nach 3 h** | **nach 8 h** | **nach 24 h** |
|---|---|---|---|---|
| **Wirkstoff/ Art** | | | | |
| **A** | 57 und 86 Schnitt: **71** | 55 und 52 Schnitt: **54** | 49 und 47 Schnitt: **48** | 42 und 49 Schnitt: **45** |
| | 70 und 74 Schnitt: **72** | 80 und 83 Schnitt: **81** | 80 und 82 Schnitt: **81** | 72 und 75 Schnitt: **74** |
| | 29 und 27 Schnitt: **28** | 25 und 27 Schnitt: **26** | 23 und 21 Schnitt : **22** | 24 und 29 Schnitt: **27** |

| | | | | |
|---|---|---|---|---|
| A: Versuche mit gelösten Feststoffen, bei denen Stent Grafts mit einer PTFE-Polymerschicht in die Lösung eingetaucht wurden. | | | | |

### Beispiel 3:

### Herstellungsverfahren (1) für FK506 beschichtete implantate:

- 10 mg FK506 werden in 3 ml Ethanol gelöst
- Unbeschichtete Stents aus Edelstahl werden unter Vakuum über Nacht bei Raumtemperatur in die Lösung getaucht.
- Dreimal 1 Minute mit Saline waschen
- Trocknen über Nacht.

### Beispiel 4:

### Herstellungsverfahren (2) für FK506 beschichtete Stent Grafts:

- Die Dosierung von FK506 kann nach Stentlänge und Durchmesser sowie nach Anwendung im Körper variieren. Hier wurde eine Dosierung von 10 - 200 µg FK506 pro cm Stentlänge verwendet.
- FK506 wird in einem kleinen Glasgefäß in Ethanol gelöst (entsprechend der gewünschten Dosierung) und die Lösung visuell auf Kristalle überprüft.
- Die nicht vorbehandelten Stent Grafts ("JOSTENT Coronary Stent Graft"), bestehend aus einer Sandwich-Konstruktion von zwei Stents aus Edelstahl mit einer PTFE-Membran, werden auf Halterungen montiert.
- Mit einer Pipette wird die entsprechende Menge an FK506-Lösung (5 bis 30 µl) auf den montierten Stent Graft aufpipettiert.
- Dieser Schritt kann nach Belieben wiederholt werden, um größere Mengen FK506 auf das Implantat aufzubringen, im Normalfall ein oder zwei Mal. Im vorliegenden Fall wurde der Vorgang einmal wiederholt.
- Nachdem der Stent Graft vollständig beladen ist, wird er vorsichtig von der Halterung geschoben.
- Nach kurzem Trocknen an der Luft (ca. 5 min), gegebenenfalls unter Wärmezufuhr, können die Stents verpackt werden.
- Alle Stents werden unter der Lupe auf Medikamentenausflockungen untersucht und gegebenenfalls verworfen.

### Beispiel 5:

### Herstellungsverfahren (3) für FK506 beschichtete Stent, die einen keramischen Überzug besitzen:

- 50 mg FK506 werden in einem Glasgefäß in 10 ml Ethanol gelöst. Von dieser Stammlösung werden bei Bedarf alle weiteren Konzentrationen durch Verdünnung hergestellt (Verdünnungen zwischen 1:1 und 1:20).
- Die Lösung wird visuell auf Kristalle überprüft.
- Die unbeladenen, mit einer Aluminiumoxid-Schicht (gemäß PCT-Anmeldung WO 00/25841, siehe auch Beispiel 7 zu keramischer Beschichtung) überzogenen Stents werden auf Halterungen montiert.
- Mit einer Injektionsspritze wird die FK506-Lösung (5 bis 50 µl) tropfenweise auf den Stent aufgetragen. Dabei sollte die Lösung über den kompletten Stent verteilt sein.
- Der beladene Stent wird vorsichtig von der Halterung genommen.
- Nach kurzem Trocknen an der Luft (ca. 5 min), gegebenenfalls unter Wärmezufuhr, können die Stents verpackt werden.
- Alle Stents werden unter der Lupe auf Medikamentenausflockungen untersucht und gegebenenfalls verworfen.

### Beispiel 6:

### Neues alternatives Herstellungsverfahren (4) (u.a. für FK506 aber auch für andere Wirkstoffe anwendbar), insbesondere zur Herstellung steriler Stents, Stent Grafts oder bzw. Polymeroberflächen-Stents :

- Es werden kleine Injektionsflaschen verwendet, die nicht sehr viel größer als der Stent sind.
- Sterile koronare Stent Grafts (CSG's) werden steril in die sterilen Injektionsflaschen gegeben.
- 0,5 ml steril-filtrierter FK506-Lösung (3.3 mg/ml in Ethanol) werden in die Flasche hinzugegeben.
- Die Flachen werden mit Gummi-Stopfen verschlossen.
- Eine sterile Injektions-Kanüle mit sterilem Filter wird durch die Mitte des Gummi-Stopfens gestochen.
- Die Flaschen werden in einem Exsikator unter Vakuum horizontal auf einem Rollengerät aufgestellt.
- Die Flaschen werden über Nacht unter Vakuum gerollt.
- Die Injektions-Nadel wird entfernt.
- Es wird nicht gespült.
- Die sterilen CSG's sind einsatzbereit.

### Beispiel 7:

### Eine Auswahl möglicher Wirkstoffe für einen wirkstoff-freisetzenden Stent, insbesondere mit mehreren Schichten, beispielsweise Polymeroberflächen-Stents bzw. Stent Grafts etc.

Die Beladungsmethoden werden als technische Ansätze unten beschrieben.

Die aufgezählten Wirkstoffe umfassen auch Derivate sowie alle Arten von Salzen, Enantiomeren, Razematen, Basen oder freien Säuren.

Besonders interessant sind hier Stents, Stent Grafts bzw. Polymeroberflächen-Stents, die mindestens einen, zwei oder drei der unten aufgezählten Wirkstoffe enthalten und entsprechend freisetzen.

Dabei werden die aufgezählten Wirkstoffe nach ihrem bevorzugten Freisetzungsprofil bzw. dem Freisetzungszeitraum in die Gruppen 1 - 3 eingeteilt.

Bevorzugt ist es dabei auch, wenn die Stents, Stent Grafts bzw. Polymeroberflächen-Stents Wirkstoffe aus verschiedenen Gruppen enthalten.

**Tabelle 8**

| **Phase I - Vasodilation (Gruppe 1)** | | |
|---|---|---|
| **Wirkstoffe, die insbesonders innerhalb der ersten 24-72 h nach Setzen des Stents freigesetzt werden.** | | |
| **Wirkstoff** | | |
| Molsidomin, Linsidomin, Natrium-Nitroprussid, Nitroglycerin, NO-donoren allgemeinl | | |
| Stimulatoren der löslichen Guanylat- Cyklase wie BAY 41-2272 (5-(Cyclopropyl-2-[1-(2-fluoro-benzyl)-1H-pyrazolo[3,4-n]pyridin-3-yl]-pyrimidin-4-ylamin) | | |
| Hydralazin | | |
| Verapamil, Diltiazem, Nifedipin, Nimodipine und andere Ca²⁺-Kanal- Blocker | | |
| Captopril, Enalapril, Lisinopril, Quinapril und andere Inhibitoren der angiotensinumwandelnden Enzyme | | |
| Losartan, Candesartan, Irbesartan, Valsartan und andere Angiotensin II-Rezeptor Antagonisten | | |

**Tabelle 9**

| **Phase II - Inhibition von Entzündung, Immunsuppression, Promotion des Zellwachstums endothelialer Zellen, Inhibition der Zell-Migration (Gruppe 2)** | | |
|---|---|---|
| **Wirkstoffe, die insbesonders innerhalb der ersten 2-21 Tage nach Setzen des Stents freigesetzt werden.** | | |
| **Wirkstoff** | | |
| Dexamethason, Betamethason, Prednison und andere Corticosteriode | | |
| 17-beta-Östradiol | | |
| FK506 (Tacrolimus) | | |
| Cyclosporin | | |
| Mycophenolsäure | | |
| VEGF, VEGF-Rezeptor Aktivatoren | | |
| Tranilast | | |
| Meloxicam, Celebrex, Vioxx und andere COX-2 Antagonisten | | |
| Indomethacin, Diclofenac, Ibuprofen, Naproxen und andere COX-1 Inhibitoren | | |
| Plasminogen-Aktivator-Inhibitor-1 und andere Serpine | | |
| Thrombin inhibitoren wie Hirudin, Hirulog, Agratroban, PPACK etc. | | |
| Interleukin-10 | | |

**Tabelle 10**

| **Phase III - Inhibition der Zell-Proliferation (Gruppe 3)** | | |
|---|---|---|
| **Wirkstoffe, die insbesonders innerhalb der ersten 14 Tage bis 3 Monate nach Setzen des Stents freigesetzt werden.** | | |
| **Wirkstoff** | | |
| Sirolimus, SDZ RAD (40-O-(2-hydroxyethyl)-Rapamycin und andere Rapamycin-Derivate | | |
| PDGF-Antagonisten | | |
| Paclitaxel | | |
| Cisplatin | | |
| Vinblastin | | |
| Mitoxantrone | | |
| Combretastatin A4 | | |
| Topotecan | | |
| Methotrexat | | |
| Flavopiridol | | |

Eine lokale Anwendung des Wirkstoffs wird durch direkte Abgabe von der wirkstoffbeladenen Oberfläche eines koronaren oder peripheren Stents erzielt. Eine wirkstoffbeiadene Oberfläche eines Stents kann durch Verwendung verschiedener technologischer Ansätze erreicht werden. Jeder dieser Ansätze kann so durchgeführt werden, daß der Wirkstoff von der Oberfläche entweder in einem kurzen (Stunden) oder einem ausgedehnten Zeitraum (Tage) freigesetzt wird. Die Freisetzungskinetik kann dadurch angepaßt werden, daß spezifische Modifikationen an der Oberfläche vorgenommen werden, z.B. hydrophobe oder hydrophile Seitenketten eines polymeren Trägers oder eine keramische Oberfläche.
- Keramische Beschichtung
   Eine Aluminiumoxid-Beschichtung (Patentanmeldungen DE 19855421, DE 19910188, WO 00/25841) mit poröser Oberfläche kann mit Wirkstoff (beispielsweise FK506 mit Mengen zwischen 10 µg und 10 mg) entweder durch Eintauchen, Aufsprühen oder eine vergleichbare Technik beladen werden. Die Dosis des Wirkstoffes hängt von der Art des Zielgefäßes und dem Zustand des Patienten ab und wird so gewählt, daß Proliferation, Migration und T-Zell-Antwort ausreichend inhibiert werden, ohne daß der Heilungsprozeß behindert wird. Der Wirkstoff kann als wässrige oder organische Lösung, beispielsweise in DMSO, DMF und Ethanol, verwendet werden. Nach dem Sprühen oder Eintauchen (unter schwachem Vakuum) wird der behandelte Stent getrocknet und der Vorgang 1 bis 5-mal wiederholt. Nach dem letzten Trockenschritt wird der Stent 1 min lang bei Raumtemperatur in Wasser oder isotonischer Saline gespült und anschließend wieder getrocknet. Der Wirkstoffgehalt kann nach Herauslösen des Wirkstoffs mit einem geeigneten Lösungsmittel durch Standardmethoden (HPLC, LC-MS) analysiert werden. Freisetzungskinetiken können unter Verwendung einer Standard-Freisetzungsmeßapparatur gemessen werden.
- PTFE Membran: Stent Graft
   Hier wird ein zu oben beschrieben identischer Ansatz verwendet. Der Wirkstoff wird in den Vertiefungen der porösen PTFE-Membran eingelagert.
- Allgemeine polymerische Beschichtung
   Verschiedene Polymere sind für eine Wirkstoffbeladung geeignet: Methacrylate-Polymere, Polyurethan-Beschichtungen, PTFE-Beschichtungen oder Hydrogel-Beschichtungen. Der Wirkstoff kann entweder auf die End-Oberfläche aufgetragen werden (s.o.) oder wird direkt der Polymerisationslösung beigegeben. In den übrigen Details entspricht dieser technische Ansatz den oben bereits beschriebenen.
- Mechanischer Ansatz
   Der mechanische Ansatz beruht auf Vertiefungen, die in die Stent-Streben mittels eines Schneid-Lasers eingebracht werden. Diese Vertiefungen können dann mit Wirkstoff gefüllt werden. Der mechanische (Vertiefungen)-Ansatz kann mit einer dünnen bioabbaubaren Beschichtung kombiniert werden, die selbst wirkstoffbeladen ist. Nach einer anfänglichen Freisetzung aus der bioabaubaren Beschichtung kann aus den wirkstoffgefüllten Vertiefungen langfristig Wirkstoff freigesetzt werden. In den übrigen Details entspricht dieser technische Ansatz den oben bereits beschriebenen.

### Beispiel 8:

### Wirkstofffreisetzung von Candesartan und Quinapril von (Polymer-) beschichteten Implantaten:

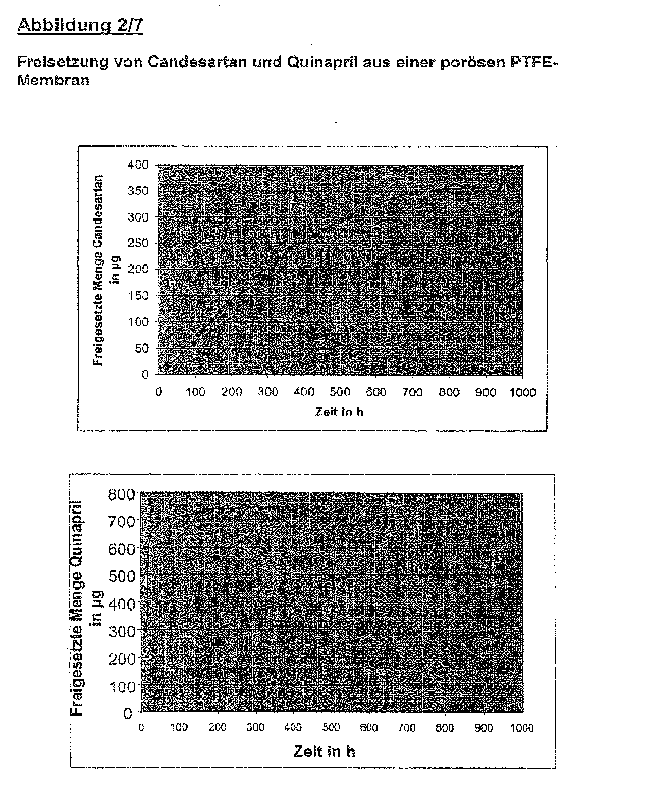

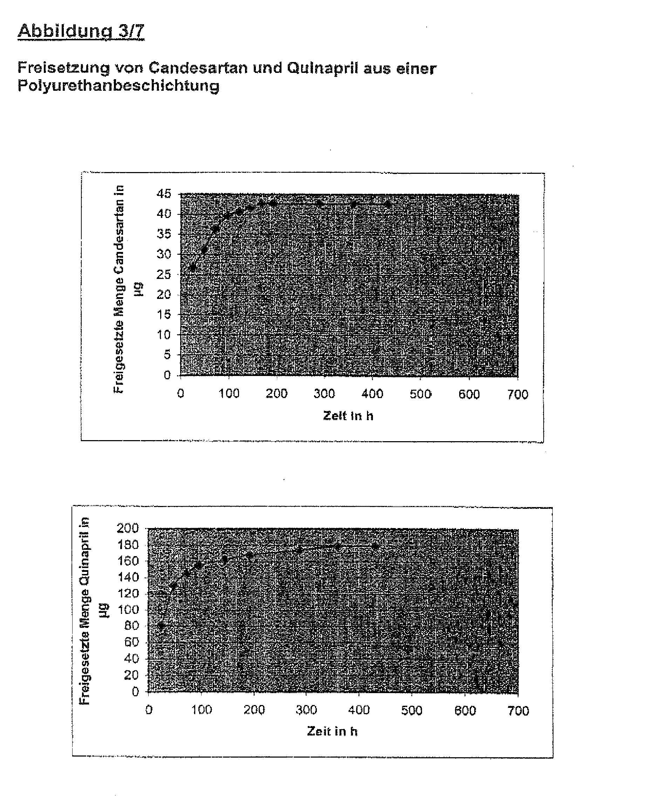

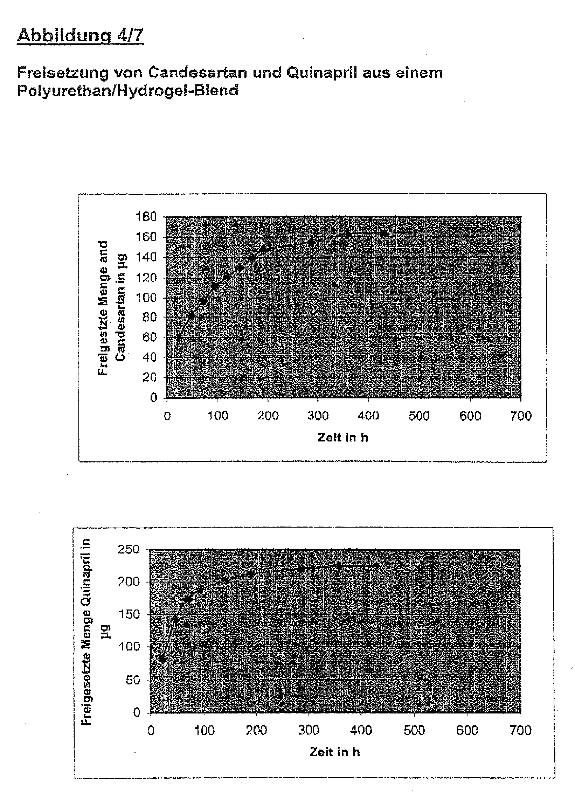

a) Stents wurden mit einer porösen PTFE-Membran ausgestattet. Diese Membran wurde anschließend mit einem Wirkstoffgemisch aus Candesartan und Quinapril beladen (je 1 mg). Die beiden Wirkstoffe werden gleichzeitig freigesetzt. Die Freisetzung wurde in PBS (phosphate buffered saline) ermittelt. Abbildung 2 zeigt die Wirkstofffreisetzung von Candesartan und Quinapril vom Stent.
b) Offenzellige Edelstahlstents wurden mit einer Polyurethanbeschichtung versehen. Ein Wirkstoffgemisch aus Candesartan und Quinapril (je 15% bezogen auf den Polyurethangehalt) wurde in eine 5%ige Lösung des Polyurethans in Dimethylacetamid eingebracht und mittels eines Sprühverfahrens auf den Stent aufgebracht. Abbildung 3 zeigt die entsprechende Wirkstofffreisetzung.
d) Offenzellige Edelstahlstents wurden mit einer Polyurethan/Hydrogelbeschichtung versehen. Ein Wirkstoffgemisch aus Candesartan und Quinapril (je 15% bezogen auf den Polymergehalt) wurde in eine 5%ige Lösung des Polymerblends in Dimethylacetamid eingebracht und mittels eines Sprühverfahrens auf den Stent aufgebracht. Abbildung 4 zeigt die entsprechende Wirkstofffreisetzung.

### Beispiel 9:

### Studie zur Freisetzungkinetik und Wirkungsweise von FK506 im Tier

Koronare Edelstahlstents (JOSTENT Flex, 16 mm) wurden mit einer keramischen Schicht aus Aluminiumoxid überzogen. Diese Beschichtung diente als Träger von FK506 (wie in Beispiel 5 sowie Beispiel 7 unter "Keramische Beschichtung" beschrieben). In diesem Fall wurden die Stents mit insgesamt 60 µg FK506 beladen. In einer Tierstudie wurden diese FK506 beschichteten Stents sowie normale Stents ohne Beschichtung in Kaninchen (n=7) in die Karotis-Arterie neuseeländischer Kaninchen implantiert. Untersucht wurde die Freisetzung von FK506 sowie die Wirkung von FK506 auf das Wachstum der Intima und die Bildung von Makrophagen und Lymphozyten.

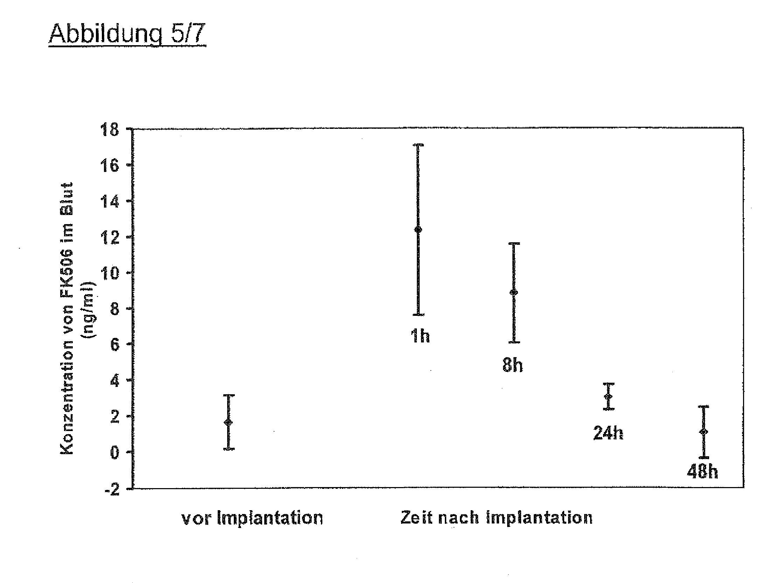

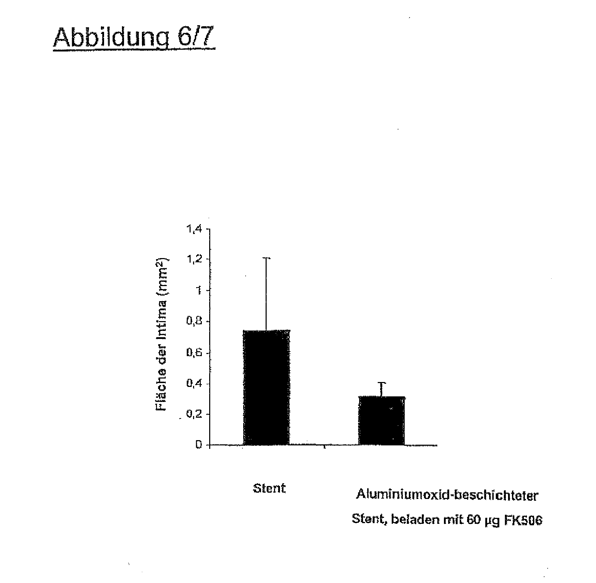

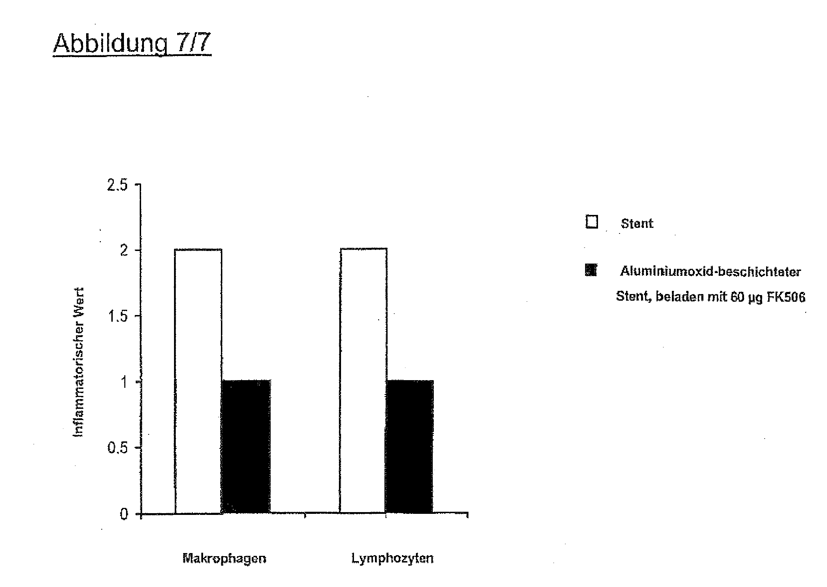

Bestimmt wurden die Freisetzung von FK506 durch HPLC-Bestimmung der Menge an FK506 im Blut der Kaninchen nach 1 h, 8 h, 24 h und 48 h. In Abbildung 5 sieht man deutlich eine sehr gute Freisetzungskinetik von FK506 über die Zeit. Die Kaninchen wurden nach 28 Tagen getötet und die implantierten Stents untersucht. Dabei wurde die Fläche der neugewachsenen Intima (Neointima) innerhalb des Stents unter dem Lichtmikroskop quantifiziert. Abbildung 6 kann man eindeutig entnehmen, daß die Bildung von Neointima bei den keramisch beschichteten Stents, die mit FK506 beladen wurden, deutlich reduziert ist im Vergleich zu normalen Stents. Bei einer Beladung mit 60 µg FK506 findet man eine Reduktion von 53%. Begleitet wurde diese Reduktion zudem durch eine spürbare Verringerung der Entzündungsherde. Wie man in Abbildung 7 erkennen kann, ist im Vergleich zum normalen Stent die Makrophagensowie die Lymphozyten-Bildung deutlich reduziert.

Der Einsatz von Stents mit einer keramischen Beschichtung, auf die FK506 aufgebracht wird, führt somit nach der Implantation zu deutlicher Reduktion von Neointima sowie von Entzündungsherden.

## Patentansprüche

1. Implantat enthaltend in chemisch kovalent oder nicht-kovalent gebundener oder physikalisch fixierter Form FK506 sowie gegebenenfalls mindestens einen weiteren Wirkstoff.

2. Implantat gemäß Anspruch 1, **dadurch gekennzeichnet, daß** es sich um ein intrakavernöses, vorzugsweise intravaskuläres, Implantat handelt.

3. Implantat gemäß einem der Ansprüche 1 oder 2, **dadurch gekennzeichnet, daß** das Implantat zur Behandlung oder Prophylaxe koronaren oder peripheren Gefäßverengungen oder -verschlüssen, insbesondere von Verengungen bzw. Stenosen oder Restenosen, vorzugsweise zur Prophylaxe von Restenose geeignet ist.

4. Implantat gemäß einem der Ansprüche 1 bis 3, **dadurch gekennzeichnet, daß** das Implantat wenigstens eine aus einem Metall oder einer Metalllegierung bestehende, homogene oder aus verschiedenen Strängen gebildete, geschlossene oder durchbrochene Schicht oder Oberfläche aufweist.

5. Implantat gemäß einem der Ansprüche 1 bis 4, **dadurch gekennzeichnet, daß** das Implantat wenigstens eine aus einem Polymer bestehende, homogene oder aus verschiedenen Strängen gebildete, geschlossene oder durchbrochene Schicht oder Oberfläche aufweist.

6. Implantat gemäß Anspruch 5, **dadurch gekennzeichnet, daß** wenigstens eine Polymerschicht ganz oder teilweise eine aus einem Metall oder einer Metalllegierung bestehende, homogene oder aus verschiedenen Strängen gebildete, geschlossene oder durchbrochene Schicht oder Oberfläche, vorzugsweise eine aus einem Metall oder einer Metalllegierung bestehende, gegebenenfalls gitterförmige Struktur bedeckt.

7. Implantat gemäß Anspruch 5, **dadurch gekennzeichnet, daß** das Implantat wenigstens eine aus einem Metall oder einer Metalllegierung bestehende, homogene oder aus verschiedenen Strängen gebildete, geschlossene oder durchbrochene Schicht oder Oberfläche und wenigstens eine aus einem Polymer bestehende, homogene oder aus verschiedenen Strängen gebildete, geschlossene oder durchbrochene Schicht oder Oberfläche aufweist.

8. Implantat gemäß Anspruch 7, **dadurch gekennzeichnet, daß** die aus einem Metall oder einer Metalllegierung bestehende Schicht oder Oberfläche eine aus einem Metall oder einer Metalllegierung bestehende, gegebenenfalls gitterförmige, Struktur ist und/oder die aus einem Polymer bestehende Schicht oder Oberfläche homogen geschlossen oder gewebt ist und/oder wasser- und/oder korpuskelundurchlässig ist und/oder die Abfolge der Schichten und Oberflächen von Außen nach Innen Metall-Polymer, Polymer-Metall, Metall-Polymer-Metall oder Polymer-Metall-Polymer ist und/oder entweder die aus einem Polymer bestehende Schicht oder Oberfläche nicht chemisch (kovalent oder nichtkovalent) mit der aus einem Metall oder einer Metalllegierung bestehenden Schicht oder Oberfläche verbunden ist oder die aus einem Polymer bestehende Schicht oder Oberfläche mittels eines Klebstoffes mit der aus einem Metall oder einer Metalllegierung bestehenden Schicht oder Oberfläche verbunden ist.

9. Implantat gemäß einem der Ansprüche 5 bis 8, **dadurch gekennzeichnet, daß** das Polymer ausgewählt ist aus Dacron; Polytetrafluorethylen (PTFE/Teflon), ausdehnbar oder nicht ausdehnbar; Polyurethan; Methacrylat-Polymeren; Hydrogel oder Hydrogel/Polyurethan-Blend, vorzugsweise aus Polytetrafluorethylen (PTFE), ausdehnbar oder nicht ausdehnbar; oder Polyurethan; insbesondere aus PTFE.

10. Implantat gemäß einem der Ansprüche 1 bis 9, **dadurch gekennzeichnet, daß** das Implantat ein Stent, ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft ist.

11. Implantat gemäß einem der Ansprüche 1 bis 10, **dadurch gekennzeichnet, daß** das Implantat mit FK506 beschichtet ist.

12. Implantat gemäß einem der Ansprüche 4, 6 oder 7 bis 10, **dadurch gekennzeichnet, daß** das Implantat einen keramischen Überzug, insbesondere aus Aluminiumoxid oder Iridiumoxid, aufweist, an den FK506 gebunden ist.

13. Implantat gemäß Anspruch 12, **dadurch gekennzeichnet, daß** der keramische Überzug ganz oder teilweise mit einem polymeren, vorzugsweise biologisch abbaubaren, Überzug bedeckt ist, an den gegebenfalls FK506 und/oder ein weiterer Wirkstoff gebunden ist oder in dem FK506 und/oder ein weiterer Wirkstoff vor Aufbringung des Überzugs gelöst wurde.

14. Implantat gemäß einem der Ansprüche 5 bis 10 oder 13, **dadurch gekennzeichnet, daß** das Implantat einen polymeren Überzug, insbesondere aus Methacrylat-Polymeren, Polyurethan, PTFE, Hydrogel oder Hydrogel/Polyurethan-Blend, insbesondere PTFE, aufweist, an den FK506 gebunden ist oder in dem FK506 vor Aufbringung des Überzugs gelöst wurde.

15. Implantat gemäß einem der Ansprüche 4, 6 oder 7 bis 10, **dadurch gekennzeichnet, daß** das Metall des Implantats mittels Laser eingebrachte Vertiefungen aufweist, die mit FK506 gefüllt sind.

16. Implantat gemäß Anspruch 15, **dadurch gekennzeichnet, daß** das mit FK506-gefüllten Vertiefungen versehene Metall oder mindestens die Vertiefungen mit einem biologisch abbaubaren Polymermaterial überzogen wird/werden, wobei gegebenenfalls FK506 an den polymeren Überzug gebunden wird oder FK506 vor Aufbringung des Überzugs in dem Polymermaterial gelöst wurde.

17. Implantat gemäß einem der Ansprüche 11 bis 16, **dadurch gekennzeichnet, daß** FK506 in Form von beladenen Nanopartikeln oder Liposomen vorliegt.

18. Implantat gemäß einem der Ansprüche 1 bis 10 herstellbar durch ein Verfahren, bei dem
a) ein wenigstens eine aus einem Metall oder einer Metall-Legierung bestehende, homogene oder aus verschiedenen Strängen gebildete, geschlossene oder durchbrochene Schicht oder Oberfläche aufweisendes Implantat gemäß einem der Ansprüche 4, 6 oder 7 bis 10, das mit einem keramischen Überzug, insbesondere aus Aluminiumoxid oder lridiumoxid, überzogen wird, eingesetzt wird
oder
b) ein wenigstens eine aus einem Polymer bestehende, homogene oder aus verschiedenen Strängen gebildete, geschlossene oder durchbrochene Schicht oder Oberfläche aufweisendes Implantat gemäß einem der Ansprüche 5 bis 10 eingesetzt wird,
oder
c) ein Implantat gemäß einem der Ansprüche 1 bis 10, das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird, eingesetzt wird oder
d) ein Implantat gemäß einem der Ansprüche 4, 6 oder 7 bis 10 mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Implantat mit einem polymerisierten oder auf der Oberfläche polymerisierenden bioabbaubaren Überzug überzogen wird, eingesetzt wird,
e) dann das Implantat gemäß a), b), c) oder d) mit einer FK506-Lösung in wäßrigem oder organischem Lösungsmittel in Kontakt gebracht wird, beispielsweise durch Beträufeln, Besprühen oder Eintauchen gegebenenfalls unter Vakuum,
f) dann gegebenenfalls das Implantat getrocknet wird, vorzugsweise bis zur Entfernung des Lösungsmittels aus Schritt e),
g) dann gegebenenfalls Schritt e) gegebenenfalls gefolgt von Schritt f) wiederholt wird, vorzugsweise mehrfach, insbesondere 1 bis 5-fach, sowie
h) gegebenenfalls anschließend das Implantat ein- oder mehrfach mit Wasser oder isotoner Saline gespült wird und
i) gegebenenfalls anschließend getrocknet wird.

19. Implantat gemäß Anspruch 18, **dadurch gekennzeichnet, daß** in Schritt e) FK506 in Alkohol gelöst wird, vorzugsweise in Ethanol, insbesondere in einer Konzentration von 0,5 - 5 g/l FK506 in Ethanol und/oder daß in Schritt e) das Implantat unter Vakuum vorzugsweise über Nacht durch Eintauchen mit einer FK506-Lösung in wäßrigem oder organischem Lösungsmittel in Kontakt gebracht wird und/oder die Schritte f) und/oder g) nicht ausgeführt werden und/oder bei Schritt h) das Implantat mehrfach mit Saline gewaschen wird und/oder in Schritt i) das Implantat über Nacht getrocknet wird.

20. Implantat gemäß Anspruch 18, **dadurch gekennzeichnet, daß** in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird, ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die FK506-Lösung bewegt wird und abschließend, vorzugsweise nach Ablauf von ca. 12 h, das dünne, vorzugsweise steril, luftdurchlässige Belüftungsrohr entfernt wird und/oder daß in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im vorzugsweise sterilen verschlossenen Glasgefäß von Schritt e) verbleibt und/oder daß die Schritte f) bis i) unterbleiben.

21. Implantat gemäß einem der Ansprüche 5 bis 10 herstellbar durch ein Verfahren, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

22. Implantat gemäß einem der Ansprüche 1 bis 21, **dadurch gekennzeichnet, daß** nach Implantierung des Implantats FK506 freigesetzt wird.

23. Implantat gemäß Anspruch 22, **dadurch gekennzeichnet, daß** die Freisetzung verzögert erfolgt.

24. Implantat gemäß Anspruch 23, **dadurch gekennzeichnet, daß** FK506 vom Implantat über einen Zeitraum von 24 h, vorzugsweise 48 h, insbesondere mehr als 96 h nach Implantierung freigesetzt wird.

25. Implantat gemäß Anspruch 23, **dadurch gekennzeichnet, daß** das FK506 vom Implantat nach Implantation
e) innerhalb von < 48 h oder
f) über mindestens 48 h, vorzugsweise über mindestens 7 Tage, insbesondere über mindestens 2 bis zu 21 Tagen freigesetzt wird,
oder daß
g) das Implantat beide Freisetzungsmuster d) und e) zeigt.

26. Implantat gemäß einem der Ansprüche 1 bis 25, **dadurch gekennzeichnet, daß** das Implantat mindestens einen weiteren Wirkstoff enthält, vorzugsweise einen pharmazeutischen Wirkstoff, insbesondere einen weiteren Wirkstoff ausgewählt aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors ;
(Gruppe 2:) Dexamethason, Betamethason, Prednison oder anderen Corticosterioden; 17-beta-Östradiol ; Cyclosporin; Mycophenol-Säure; VEGF, VEGF-Rezeptor Aktivatoren; Tranifast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol,
Actinomycin D; Rheopro/Abciximab oder Probucol.

27. Implantat gemäß Anspruch 26, **dadurch gekennzeichnet, daß**, wenn der weitere Wirkstoff ausgewählt ist aus Gruppe 1, dieser vom Implantat innerhalb der ersten 24 - 72 h nach Implantation freigesetzt wird und/oder, wenn der weitere Wirkstoff ausgewählt ist aus Gruppe 2, dieser vom Implantat innerhalb der ersten 48 h - 21 Tagen nach Implantation freigesetzt wird und/oder, wenn der weitere Wirkstoff ausgewählt ist aus Gruppe 3, dieser vom Implantat innerhalb von 14 Tagen bis 3 Monaten nach Implantation freigesetzt wird.

28. Verfahren zur Herstellung eines Implantats gemäß einem der Ansprüche 1 bis 25 mit folgenden Schritten:
a) ein wenigstens eine aus einem Metall oder einer Metall-Legierung bestehende, homogene oder aus verschiedenen Strängen gebildete, geschlossene oder durchbrochene Schicht oder Oberfläche aufweisendes Implantat gemäß einem der Ansprüche 4, 6 oder 7 bis 10, das mit einem keramischen Überzug, insbesondere aus Aluminiumoxid oder lridiumoxid, überzogen wird,
oder
b) ein wenigstens eine aus einem Polymer bestehende, homogene oder aus verschiedenen Strängen gebildete, geschlossene oder durchbrochene Schicht oder Oberfläche aufweisendes Implantat gemäß einem der Ansprüche 5 bis 10,
oder
c) ein Implantat gemäß einem der Ansprüche 1 bis 10, das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird,
oder
d) ein Implantat gemäß einem der Ansprüche 4, 6 oder 7 bis 10 mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Implantat mit einem polymerisierten oder auf der Oberfläche polymerisierenden vorzugsweise bioabbaubaren Überzug überzogen wird,
wird eingesetzt,
e) dann wird das Implantat gemäß a), b), c) oder d) mit einer FK506-Lösung in wäßrigem oder organischem Lösungsmittel in Kontakt gebracht, beispielsweise durch Beträufeln, Sprühen oder Eintauchen, gegebenenfalls unter Vakuum,
f) dann wird gegebenenfalls das lmplantat getrocknet, vorzugsweise bis das Lösungsmittel aus Schritt e) verdampft ist,
g) dann wird gegebenenfalls Schritt e) gegebenenfalls gefolgt von Schritt f), vorzugsweise mehrfach, insbesondere 1 bis 5-fach, wiederholt sowie
h) gegebenenfalls wird anschließend das Implantat ein- oder mehrfach mit Wasser oder isotoner Saline gespült und
i) gegebenenfalls wird anschließend getrocknet.

29. Verfahren gemäß Anspruch 28, **dadurch gekennzeichnet, daß** in Schritt e) FK506 in Alkohol gelöst wird, vorzugsweise in Ethanol, insbesondere in einer Konzentration von 0,5 - 5 g/l FK506 in Ethanol und/oder daß in Schritt e) das Implantat unter Vakuum vorzugsweise über Nacht durch Eintauchen mit einer FK506-Lösung in wäßrigem oder organischem Lösungsmittel in Kontakt gebracht wird und/oder daß die Schritte f) und/oder g) nicht ausgeführt werden und/oder daß bei Schritt h) das Implantat mehrfach mit Saline gewaschen wird und/oder daß in Schritt i) das Implantat über Nacht getrocknet wird.

30. Verfahren gemäß Anspruch 28, **dadurch gekennzeichnet, daß** in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die FK506-Lösung in Bewegung gehalten wird und abschließend vorzugsweise nach Ablauf von ca. 12h das dünne, vorzugsweise steril, luftdurchlässige Belüftungsrohr entfernt wird und/oder daß in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im sterilen verschlossenen Glasgefäß von Schritt e) verbleibt und/oder daß die Schritte f) bis i) unterbleiben.

31. Verfahren zur Herstellung von Implantaten gemäß einem der Ansprüche 5 bis 10, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

32. Verfahren zur Herstellung mit Wirkstoffen beschichteter Implantate mit folgenden Schritten:
a) das Implantat wird, vorzugsweise steril, in ein vorzugsweise steriles Gefäß mit einem sich nach Ende einer Perforation verschließenden, perforierbarem Verschluß, beispielsweise in eine Injektions-Flasche eingebracht
b) vorzugsweise sterile Wirkstofflösung, vorzugsweise in einem organischen Lösungsmittel mit geringem Dampfdruck, insbesondere in Alkohol wie Ethanol oder Methanol, wird in das Gefäß eingebracht.
c) das Gefäß wird mit dem sich nach Ende einer Perforation verschließenden, perforierbarem Verschluß verschlossen,
d) ein dünnes, vorzugsweise steril, luftdurchlässiges Belüftungsrohr, beispielsweise eine Kanüle, wird perforierend durch den Verschluß gesteckt wird,
e) gegebenenfalls wird ein Vakuum angelegt, wobei vorzugsweise die Wirkstoff-Lösung bewegt wird,
f) abschließend, vorzugsweise nach Ablauf von ca. 12h, wird das dünne, vorzugsweise steril, luftdurchlässige Belüftungsrohr entfernt und
g) gegebenenfalls das Implantat bis zum Einsatz im vorzugsweise sterilen verschlossenen Glasgefäß von Schritt a) belassen.

33. Verfahren gemäß Anspruch 32, **dadurch gekennzeichnet, daß** das Implantat von Schritt a) mindestens eine metallische durchbrochene oder geschlossene Oberfläche oder Schicht aufweist, eine keramische Beschichtung aufweist, eine polymere Beschichtung aufweist und/oder mindestens eine polymere, durchbrochene oder geschlossene Oberfläche oder Schicht aufweist.

34. Verfahren gemäß einem der Ansprüche 32 oder 33, **dadurch gekennzeichnet, daß** das Implantat ein Stent, Stent Graft, Graft, Graft Verbinder, Polymeroberflächen-Stent oder Katheter ist.

35. Verfahren gemäß einem der Ansprüche 32 bis 34, **dadurch gekennzeichnet, daß** der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cydopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavapiridol;
Actinomycin D; Rheopro/Abciximab oder Probucol
insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); VEGF, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Mitoxantron; Combretastatin A4; Flavopiridol.

36. Verwendung eines Implantats gemäß einem der Ansprüche 1 bis 27 zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

37. Verwendung von FK506 zur Beschichtung oder zur Herstellung eines Implantats zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

38. Verwendung gemäß Anspruch 37, **dadurch gekennzeichnet, daß** das Implantat ein Stent, ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft bzw. ein Polymeroberflächen-Stent ist.

39. Verwendung gemäß einem der Ansprüche 37 oder 38, **dadurch gekennzeichnet, daß** das FK506 am Implantat so gebunden oder angebracht ist, daß es nach Implantierung vom Implantat, vorzugsweise verzögert, freigesetzt wird.

40. Verwendung von FK506 zur Behandlung oder Prophylaxe von koronaren oder peripheren Gefäßverengungen oder-verschlüssen, insbesondere von Verengungen bzw. Stenosen oder Restenosen, vorzugsweise zur Prophylaxe von Restenose.

41. Polymeroberflächen-Stent enthaltend in chemisch kovalent oder nicht-kovalent gebundener oder physikalisch fixierter Form mindestens einen physiologisch und/oder pharmazeutisch wirksamen Wirkstoff.

42. Polymeroberflächen-Stent gemäß Anspruch 41, **dadurch gekennzeichnet, daß** der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY41-2272 (5-(Cyclopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); 17 -beta-Östradiol; Cyclosporin, Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol
insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1H-pyrazolo[3,4-n]pyridin-3-yl]-pyrimidin-4-ylamine); Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); VEGF, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Mitoxantron; Combretastatin A4; Flavopiridol;
und/oder daß der Polymeroberflächen-Stent mindestens zwei, vorzugsweise 2 oder 3 physiologisch und/oder pharmazeutisch wirksame Wirkstoffe ausgewählt aus einer der Gruppen 1 bis 3 enthält, vorzugsweise maximal einen Wirkstoff aus einer Gruppe.

43. Polymeroberflächen-Stent gemäß Anspruch 42, **dadurch gekennzeichnet, daß** wenn der weitere Wirkstoff ausgewählt ist aus Gruppe 1, dieser vom Implantat innerhalb der ersten 24 - 72 h nach Implantation freigesetzt wird und/oder, wenn der weitere Wirkstoff ausgewählt ist aus Gruppe 2, dieser vom Implantat innerhalb der ersten 48 h - 21 Tagen nach Implantation freigesetzt wird und/oder, wenn der weitere Wirkstoff ausgewählt ist aus Gruppe 3, dieser vom Implantat innerhalb von 14 Tagen bis 3 Monaten nach Implantation freigesetzt wird.
